# EUROPEAN PATENT SPECIFICATION

(11) **EP 3 638 104 B1**
(45) Date of publication and mention of the grant of the patent: **01.04.2026**
(21) Application number: 18735383.4
(22) Date of filing: 14.06.2018
(51) Int. Cl.: A61B 5/00, A61B 5/01, A61B 5/145, A61B 5/0205, A61B 5/024, A61B 5/20, A61B 5/103

(54) **MEDICAL DEVICES**
MEDIZINISCHE GERÄTE
APPAREILS MÉDICAUX

(30) Priority: 14.06.2017 GB 201709435
(43) Date of publication of application: 22.04.2020
(73) Proprietor: Bevan, Heba, Bromley, Greater London BR1 4JE (GB)
(72) Inventor: Bevan, Heba, Bromley, Greater London BR1 4JE (GB)
(74) Representative: Marks & Clerk LLP
(86) International application number: PCT/GB2018/051647
(87) International publication number: WO 2018/229498

(56) References cited:
- EP-A1- 2 526 856
- WO-A1-2009/138979
- WO-A1-2009/138979
- WO-A1-2015/173726
- WO-A1-2015/173726
- WO-A1-2017/009878
- WO-A1-2017/096094
- GB-A- 2 437 549
- GB-A- 2 437 549
- US-A1- 2005 256 428
- US-A1- 2005 256 428
- US-A1- 2008 275 366
- US-A1- 2008 275 366
- US-A1- 2011 265 576
- US-A1- 2011 265 576
- US-A1- 2013 053 728
- US-A1- 2013 053 728
- US-A1- 2016 029 942
- US-A1- 2016 029 942
- US-A1- 2017 135 622
- US-A1- 2017 135 622
- SANCHETI NARENDRA: "Presentation on understanding uroflowmetry Volume 3 Issue 6 -2016", UROLOGY & NEPHROLOGY OPEN ACCESS JOURNAL, 8 December 2016 (2016-12-08), XP055805380, Retrieved from the Internet <URL:https://medcraveonline.com/UNOAJ/UNOAJ-03-00104.pdf> [retrieved on 20210518]
- ANONYMOUS: "Uroflowmetry System - Santron Compact Uro Flowmeter Manufacturer from Pune.", 19 October 2016 (2016-10-19), pages 1 - 3, XP055805495, Retrieved from the Internet <URL:http://web.archive.org/web/20161019181200/https://www.santronmeditronic.com/uroflowmetry-system.html> [retrieved on 20210518]
- SANCHETI NARENDRA: "Presentation on understanding uroflowmetry Volume 3 Issue 6 -2016", UROLOGY & NEPHROLOGY OPEN ACCESS JOURNAL, 8 December 2016 (2016-12-08), XP055805380, Retrieved from the Internet <URL:https://medcraveonline.com/UNOAJ/UNOAJ-03-00104.pdf> [retrieved on 20210518]
- ANONYMOUS: "Uroflowmetry System - Santron Compact Uro Flowmeter Manufacturer from Pune.", 19 October 2016 (2016-10-19), pages 1 - 3, XP055805495, Retrieved from the Internet <URL:http://web.archive.org/web/20161019181200/https://www.santronmeditronic.com/uroflowmetry-system.html> [retrieved on 20210518]
- XIAO LI ET AL: "Digital Health: Tracking Physiomes and Activity Using Wearable Biosensors Reveals Useful Health-Related Information", PLOS BIOLOGY, vol. 15, no. 1, 12 January 2017 (2017-01-12), pages e2001402, XP055500401, DOI: 10.1371/journal.pbio.2001402
- ANONYMOUS: "Temperature curves", 28 May 2017 (2017-05-28), XP055500827, Retrieved from the Internet <URL:http://web.archive.org/web/20170528210050/http://medicalency.com/temperature.htm> [retrieved on 20180820]
- DAVID J DREWRY ET AL: "Body temperature patterns as a predictor of hospital-acquired sepsis in afebrile adult intensive care unit patients: a case-control study", POLAR RECORD, 12 September 2013 (2013-09-12), pages 355 - 357, XP055500421, Retrieved from the Internet <URL:https://www.ncbi.nlm.nih.gov/pmc/articles/PMC3906745/pdf/cc12894.pdf> [retrieved on 20180817], DOI: 10.1017/S0032247400017101

## Description

### FIELD OF THE INVENTION

This invention relates to medical devices for detecting infection, and to related computer program code.

### BACKGROUND TO THE INVENTION

Typically when at home and an infection is suspected a measurement is made of body temperature. If this is elevated then potentially an infection is present and a visit to a doctor or hospital may be made. However, it would be useful to be able to improve upon such approaches, in particular for children and old people.

Sancheti Narendra: "Presentation on understanding uroflowmetry Volume 3 Issue 6 - 2016", Urology & Nephrology Open Access Journal, 8 December 2016, discloses a non-invasive system that measures the flow rate and volume of a urine stream. WO 2015/173726 A1 discloses an apparatus for measuring at least one measure related to urination of an individual such as urine flow and/or urine quantity. WO 2009/138979 A1 discloses a monitoring system and method for use in monitoring a condition of a patient's urinary system. US 2011/265576 A1 discloses a uroflowmeter attachable to a toilet capable of diagnosing a BPH by checking the urinary flow rate of a patient. US 2017/135622 A1 discloses a urine flow meter, a water wheel which is caused to rotate by a flow of urine from a urine flow passage tube and is provided at a lower end side of the urine flow passage tube. US 2016/0029942 A1 discloses flow rate measurement devices and systems for urine analysis to determine BPH or USD generally comprising a recording unit, an analysis unit and a transmission unit. US 2008/0275366 A1 discloses systems for and methods of assessing lower urinary tract function from urinary flow data via sound analysis and user-provided information regarding the lower urinary tract symptoms of frequency, urgency and urge incontinence.

### SUMMARY OF THE INVENTION

The invention is defined by the appended claims.

The infection sensing system may make use of chemical and/or gas sensors to identify the presence of an infection. Accordingly, in some examples the sensors further comprise one or more of a chemical sensor or a gas sensor, and the processor is configured to identify that a combination of (i) a greater than threshold temperature fluctuation in said body temperature, (ii) a greater than threshold heart rate, optionally (iii) a greater than threshold skin moisture level, and (iv) a greater than threshold concentration of chemical indicative of a medical condition, are present for a duration greater than said threshold time duration.

The chemical indicative of a medical condition may be a by-product of a medical condition, for instance, an expression of a gene associated with cancer, or glucose or one or more ketones associated with diabetes. The chemical sensor may be configured to measure one or more chemicals on the skin. The gas sensor may be configured to measure one or more chemicals in the air around the system (adjacent to the skin).

In examples a supervised or unsupervised machine learning algorithm may be employed to process one or more of body temperature data, heart rate data, and skin moisture data (all as described further below). A supervised machine learning algorithm may operate in combination with data indicating an actual infection (for example from a physician), to allow the machine learning algorithm to provide a prediction of infection. The supervised machine learning algorithm may be used to train a neural network. An unsupervised machine learning algorithm may operate on the input data to learn patterns in the data indicative of infection without the need for feedback from a physician. Optionally the machine learning may be performed in hardware and/or software in the sensor device, for example on a CPU of the sensor device; alternatively the algorithm may be partially or wholly implemented elsewhere, for example in a base station or on a remote server. Accordingly, measurements from the infection sensing system may be output to an external processing system to analyse the measurements to determine whether an infection is present in accordance with the infection identification steps described herein.

Examples of the system may include a processor in combination with working memory and non-volatile memory including memory storing processor control code to implement the above described functions. The processor(s) employed in the device may operate under the control of stored program code, or may comprise dedicated hardware implemented in electronic circuitry, or may comprise a combination of some dedicated hardware modules and some systems under program control. Additionally or alternatively, however, the data processing described above may be implemented partially or wholly in dedicated hardware such as a programmable gate array and/or ASIC (application specific integrated circuit). As the skilled person will be aware, the functionality of such a device may be distributed between multiple hardware elements in wired or wireless communication with one another; some or all processing may be performed in hardware, some or all processing may be performed in software.

The disclosure further provides processor control code to implement the above-described devices and methods, for example on a general purpose computer system or on a mobile device, or on a digital signal processor (DSP). The code is provided on a non-transitory physical data carrier such as a disk, CD- or DVD-ROM, programmed memory such as non-volatile memory (eg Flash) or read-only memory (Firmware). Code (and/or data) to implement examples may comprise source, object or executable code in a conventional programming language (interpreted or compiled) such as C, or assembly code, or code for a hardware description language. As the skilled person will appreciate such code and/or data may be distributed between a plurality of coupled components in communication with one another.

### Urine-flow based diagnostic systems

According to the invention, it is provided a urine-flow based diagnostic system, as defined by claim 1, the system comprising: a sensor device for use in a toilet bowl or urinal so as to intersect a stream of urine, the sensor device comprising a urine stream flow sensor; and a processor coupled to said urine stream flow sensor; and wherein the processor is configured to: determine, from said urine stream flow sensor, a flow rate parameter dependent on a sensed urine flow; and responsive to said flow rate parameter, store and/or output data indicating presence of a potential medical condition.

According to the invention, the device is configured to provide distinction between the presence of a prostate condition and an infection.

Embodiments of such a device are particularly useful in identifying potential urinary tract infections (UTIs) although they are not limited to detecting infections of this type. For example the device can also be used to detect a prostate condition such as prostate cancer. In practice there are other conditions which can mimic the presence of infection or prostate cancer (for example Benign Prostatic Hyperplasia may mimic prostate cancer). The device does not diagnose a condition, but rather provides an indication of a potential problem which should then be followed up with a proper medical examination.

Detecting a potential UTI in the elderly is difficult because their immune system may not mount an effective response and thus they may not exhibit fever; instead the symptoms may be similar to dementia. Nonetheless the elderly are vulnerable to UTIs which, if left untreated, can cause serious complications such as kidney damage/failure and sepsis. It is also difficult to identify UTIs in children.

The system may comprise just the sensor device, which may include the processor, and/or the sensor device may be wired or wirelessly coupled to the or another processor in a separate device, for example a mobile device or base station. Additionally or alternatively the system may include a remote server, for example in the cloud. The base station and/or remote server may perform additional processing on processed data derived from the sensor device, for improved accuracy.

In embodiments a supervised or unsupervised machine learning algorithm may be employed to process one or more of urine flow rate data, urine flow/pressure data, flow peak count data, flow peak timing data, and urine colour data (all as described further below). A supervised machine learning algorithm may operate in combination with data from a physician on an actually diagnosed condition (to supervise the learning), to allow the machine learning algorithm to provide a prediction of one or more medical conditions. The supervised machine learning algorithm may be used to train a neural network. An unsupervised machine learning algorithm may operate on the input data to learn patterns in or classify the data which patterns or classification(s) are indicative of one or more medical conditions without the need for feedback from a physician. Optionally the machine learning may be performed in hardware and/or software in the sensor device, for example on a CPU of the sensor device.

The flow rate parameter may comprise raw or processed data from one or more pressure sensors; if multiple pressure sensors are employed an average of the sensors or of a selected one or more of the sensors may be used. In embodiments the urine flow rate is determined from sensed pressure by a applying calibration parameter; this may depend upon the sensor/sensor plate geometry and may be determined by experiment for a particular sensor or sensor combination. Examples of sensor configurations are described later.

Embodiments of the device we describe detect a potential UTI or other condition from measuring flow of the urine stream. An infection or other medical condition may be detected by a reduction in urine flow rate, for example, to less than 10, 8 or 5 ml/s - a figure which is surprisingly relatively independent of age. More particularly, however, intermittent flow rate appears to be characteristic of infection, and the duration between periods of flow appears to relate to the degree of infection, longer periods being associated with a greater level of infection.

Embodiments of the device are configured to detect, from the flow rate parameter, when the urine flow is intermittent. For example, in embodiments this may be achieved by detecting a greater than threshold fluctuation in flow rate, or of a sensed parameter dependent upon the flow rate. Additionally or alternatively, periods of relatively higher and lower flow rate may be distinguished, for example by comparing the flow rate with a threshold (which may optionally exhibit hysteresis), flow rates (or rate-sensing parameters) having a value less than this threshold denoting reduced or ceased flow and flow rates higher than this value denoting adequate flow. For example in such instances the threshold is chosen to be below the reduced flow rate expected when infection is present, so as to distinguish between reduced flow rate and close to stopped flow. Detection of such intermittent flow may be used to store and/or output data indicating an infection or other condition. In embodiments the degree of infection may be determined, for example by classifying the duration between periods of intermittent flow into one of a plurality of categories determining one of a (corresponding) categories of level of infection.

Preferred embodiments of the system process the flow rate parameter (for example pressure sensor data) to distinguish peaks in the parameter. Typically this involves some filtering of the data and a implementation of peak detection procedure, an example of which is described later.

The system may be configured to identify a final part of the flow from a flow pattern or flow rate of the flow, and to disregard this. In embodiments peaks are detected down to a threshold maximum peak height, for example 0.2 ml/sec flow rate. In embodiments the final one or two peaks are then discarded.

According to the invention, a count of the number of peaks is identified. In
embodiments one or more of the following items of information may then be derived: a maximum urine flow rate (or pressure) over a peak (or some value representing this); an interval between one or more peaks (denoted T1 later); and a peak duration (denoted T3 later). A peak duration may be determined from a duration between corresponding flow rates, for example minimum flow rates, to either side of the peak.

Preferably the system also determines a time duration of a period of reduced or substantially zero flow between one or more peaks (denoted T2 later). This may be determined from a duration between corresponding flow rates to either side of a minimum between adjacent peaks. The corresponding flow rates may be a threshold level above the minimum flow rate; that is the T2 duration may be defined as the time between flow rates defined by the minimum flow rate plus a delta. In this way the time duration between peaks may be meaningfully defined whether or not the flow rate reduced to zero between peaks.

The skilled person will appreciate that where references are to flow rate, a value dependent upon or proportional to flow rate, for example a sensed pressure (or value dependent thereupon) may likewise be employed. Where it is desired to employ a peak-dependent timing value as described above, where multiple peaks are present an average value may be employed.

The system may be configured to identify when a peak flow rate, more particularly the flow rate of the initial or highest detected peak, is less than a threshold value, for example 20 ml/sec, 15 ml/sec or, preferably, 10 ml/sec, to identify the presence of a potential medical condition. Optionally two threshold flow rates may be employed, a first, higher threshold to detect the potential presence of a prostate condition such as prostate cancer, and a second, lower threshold to detect the potential presence of an infection. The higher threshold may be, for example 20 ml/sec, 15 ml/sec or, preferably, 10 ml/sec; the lower threshold may be 15 ml/sec, 10 ml/sec or, preferably, 5 ml/sec. Optionally the flow rate may be integrated to determine a total volume flow.

Depending upon the use case, multiple total flow measurements may be combined (where, say, the measurements relate to the same individual), for example to determine a morning or afternoon or daily total volume flow.

Some preferred embodiments of the system include an optical sensor to detect a "colour" of the urine, that is to distinguish between clear and dark urine. Such a sensor may comprise, for example, a light source (eg LED) and detector. In broad terms this allows embodiments of the system to detect the presence of blood in the urine. However, as previously noted, embodiments of the system do not provide a diagnosis but merely indicates a need for further investigation - for example eating beetroot can give a false positive. In preferred embodiments the colour detection is combined with other detected signals of an infection as described above and below; in embodiments multiple such signals are required for the system to indicate the presence of a potential infection.

According to the invention, the system determines the number of peaks and uses this to identify the presence of a potential medical condition. More particularly, the presence of multiple peaks indicates a potential medical condition, and a count of the number of peaks can distinguish between a prostate condition such as prostate cancer and an infection - one, two or a small number of peaks is indicative of a prostate condition such as prostate cancer, particularly in combination with other indicators, such as flow rate, as described herein; whilst two or more than two peaks is indicative of an infection. (This is particularly where the final one or two small peaks are disregarded). In broad terms, where an infection is present there are more, smaller peaks than for potential prostate cancer. Where an infection and a prostate condition such as prostate cancer are both present the number of peaks tends to correspond to that for a prostate condition rather to that for infection.

Experimental work has determined that when an infection is present the duration of a peak, T3, may be below a threshold value; and/or T2 may be greater than a threshold value (for example greater than 0.5 secs or greater than 1 second); and/or T1 (as measured, say, between the first two peaks, or as an average over peaks) may be lower than a predetermined threshold value.

Experimental work has determined that when a prostate condition such as prostate cancer is present T3, may be greater than a threshold value; and/or T2 may be less than a threshold value (for example less than 0.5 secs or substantially zero); and/or T1 (as measured, say, between the first two peaks, or as an average over peaks) may be greater than a predetermined threshold value.

It will be appreciated that the differences in the responses of one or more of these timings may be employed to differentiate between an infection and a prostate condition. Optionally a ratio of T2 to T3, such as T2/T3, may also be used to identify changes in T2 and/or T3 and/or to determine the presence of a condition and/or to differentiate between infection and a prostate condition (T2/T3 is higher for a prostate condition than for an infection).

Optionally the timing and/or flow rate data and/or peak count data may be used to determine the potential degree of a medical condition such as an infection

The system may analyse one or more chemicals in the urine or in gas from the urine. In further embodiments: the sensor device further comprises a chemical sensor, and said processor is further configured to identify presence of the potential medical condition responsive to a detected concentration of a chemical indicative of a medical condition within said urine; and/or said sensor device further comprises a gas sensor and said processor is further configured to identify presence of the potential medical condition responsive to a detected concentration of a chemical indicative of a medical condition in gas deriving from said urine.

Certain chemicals and smells are indicative of a medical condition. The chemical sensor may be configured to detect one or more gene products (e.g. RNA or protein) of genes associated with cancer. For instance, prostate-specific antigen (PSA) is a protein produced exclusively by prostate cells. PSA is a glycoprotein enzyme. There is a blood test to measure PSA level in men. This may help to detect early prostate cancer. Having said this, the blood test is not accurate.

An increased level of PSA increases the chance that the subject has prostate cancer. 13% of men over 55 have a PSA level of greater than 4 ng/ml. Having said this, an increased level of PSA does not necessarily mean that the subject has prostate cancer. An elevated level can also be due to other conditions, such as benign enlargement of the prostate (BPH), a urinary tract infection or a prostate infection.

Accordingly, by detecting an increased level of PSA within urine (or within gas deriving from the urine), embodiments may be able to identify the presence of a medical condition for further investigation by a trained medical professional.

Other chemicals that may be detected include expressions of TMPRSS2:ERG or PCA3, both of which are indicative of prostate cancer, and/or an increased level of ketones or glucose within urine, which can be indicative of diabetes.

The systems described herein may indicate the detection of a medical condition when a concentration of the chemical is detected that exceeds a predefined threshold.

The chemical or gas sensors may be configured to identify chemicals that are not indicative of a medical condition, but that may cause a false positive in the system. For instance, the presence of beetroot products (e.g. betalain pigments) within urine can cause discolouration that the system may mischaracterise as blood. Accordingly, the system may be configured to detect one or more pigments within the urine that are not associated with a medical condition in order to avoid mischaracterisation. Upon identification (e.g. upon measuring a concentration that exceeds a predefined threshold), the system may inhibit any response that derives from a measurement of the colour of the urine, or may compensation for any colour measurement to account for the measured pigment. The chemical and gas sensors may also identify an increased concentration of the urine, which may be indicative of dehydration. This may be further indicated by an increased concentration of sulphur within the urine or within gas emanating from the urine.

Optionally the system may include a local alert device such as a display device or alarm, which may be located in a bathroom (restroom), for example adjacent a toilet, to alert a user to the presence of a potential medical condition.

In embodiments the flow sensor comprises a plurality of spatially distributed flow sensors, in separate housings and/or in multiple housings (sensor enclosures). One device may be in wired or wireless communication with one or more other devices, to enable sensed data to be combined, for example averaged.

In one embodiment a sensor device may comprise an enclosure with a sensing surface comprising a moveable plate or membrane mounted on a set of one or more pressure sensors to provide distributed flow sensors. Signals from multiple sensors may be combined by averaging or the like. A pressure sensor may comprise an accelerometer in combination with a spring, to detect movement of the membrane or plate. Embodiments of the device determine flow rate or a proxy for flow rate such as pressure or an accelerometer signal, as time series data.

In one embodiment a sensor device may comprise a ring-shaped enclosure mounting a pressure-sensing surface comprising a plate or membrane having a set of apertures through which urine may pass. Such a device may be mounted across the neck of a toilet bowl (and may additionally be used to sense the passing of a kidney stone). Such a device may be used in combination with one or more other sensor devices placed at one or more other locations in a toilet bowl, for example on a forward or rearward surface of the bowl. Optionally the ring-shaped enclosure may be suspended from one or more other sensor devices which may themselves be fastened, for example glued onto the toilet bowl. The sensor device with the ring-shaped enclosure may be attached to one or more other sensor devices, for example by means of a releasable, for example magnetically fastened, attachment such as an attachment line or connection. Signals from these multiple sensing devices may be combined.

It has been found that conventional flushing systems in toilets and urinals do not always completely clean away contaminants and waste from the sensors after use. This can lead to inaccurate measurements when the system is next used. For instance, the system may incorrectly detect blood within the urine if some blood is left over from the previous use.

In one embodiment, the system further comprises a cleaning system comprising a reservoir configured to hold fluid and a release mechanism for releasing at least some of the fluid to clean at least part of the sensor device. This allows the system to clean contaminants and waste away from the sensor device after use. Cleaning the sensor device may comprise flushing away, dissolving and/or chemically breaking down (e.g. chemically cleaning away) contaminants. The fluid may by cleaning fluid. The fluid may comprise any fluid suitable for washing away contaminants or waste, such as water, bleach, or solvent(s). The reservoir may be built into the system which, in turn, may be retrofitted to a toilet or urinal. Accordingly the flushing mechanism may be in addition to a flushing mechanism of the toilet or urinal.

According to an embodiment the processor is configured to control the release mechanism to release the fluid after urine has been detected. The system may detect when a person has finished urinating and release the cleaning fluid to clean the system in order to prepare the system for future use. The system may determine that a person has finished urinating when no urine flow has been detected for a predetermined period of time or when an end of use input has been triggered. This end of use input may be the toilet or urinal being flushed (e.g. the detection of water) or through an input from the user such as via a button, pull-chord, or via an input from a device connected to the system (e.g. a mobile phone).

According to a further embodiment the system comprises a fluid collection mechanism configured to collect the fluid over one or more sensing apparatus of the system after the cleaning fluid has been released from the reservoir. This may be in the form of an actuating barrier that may be raised so that fluid may be collected over the one or more sensing apparatus, and that may subsequently be lowered in order to release the fluid. The fluid collection mechanism may release the fluid after a predetermined period of time, or after the one or more sensors indicate that they are sufficiently clean (e.g. that they are within a predefined range of normal operating parameters). This provides the fluid additional time to clean the one or more sensors. The actuating barrier may be the movable plate mentioned above. The one or more sensors may comprise a colour sensor, a chemical sensor and/or a gas sensor.

The time at which the urination takes place may also indicate a medical condition. For instance, an increase in the frequency of urination (the frequency that the subject needs to urinate) and/or an increase in the frequency of urination during a period of the day or night may indicate a medical condition. For instance, increased frequency of urination at night may be indicative of a urinary tract infection, bladder infection or diabetes (among other conditions). Accordingly, in one embodiment the processor is configured to record a time at which the urine flow is detected and, responsive to one or more of an increased frequency of urination or an increased frequency of urination during a predetermined period of the day-night cycle, store and/or output data indicating the presence of a medical condition.

Multiple different users may make use of the system. Accordingly, it may be advantageous to reset the system so that measurements are not shared between different users. According to a further embodiment the system comprises an input means and the processor is configured to identify the presence of a new user in response to an input and, in response to the identification of the new user, establish a new set of measurements for the new user. The input may be the detection of a flush by the user (e.g. via the flow sensor) or via an input from the user such as from a button, pull-chord, or via a device connected to the system (e.g. a mobile phone).

Where multiple users use the system, it can be advantageous to track each user's measurements across multiple uses. This can allow the system to build up a profile for the user so that anomalous results can be detected over time. According to an embodiment the processor is configured to identify the new user in response to receipt of a unique user identifier and to associate any subsequent measurements and identifications of medical conditions with the unique user identifier until the presence of another user is identified. The unique user identifier may be received via an input from a radio frequency identification (RFID) reader configured to read an RFID tag or magnetic reader configured to read a magnetic tag.

The infection sensing systems, that do not form part of the claimed invention, and the urine-flow based diagnostic systems, according to the invention, described herein may be configured to communicate with each other, e.g. via wireless communication. This can allow the systems to share measurements to allow more accurate identification of medical conditions. According to an example there is provided an infection sensing system further as described herein further comprising a wireless communication module, wherein the processor is configured to trigger one or more measurements by one or more of the sensors in response to an input from a urine-flow based diagnostic system as described herein indicating that the user of the infection sensing system is urinating. This allows the systems to work together to take additional measurements during urination. These additional measurements may include one or more of heart rate, skin temperature, skin moisture, chemical measurements and gas measurements.

Where multiple sensors are present the system (code) may be configured to distinguish between genders flow dependent upon a sensed spatial distribution of the urine flow. For example where sensors are located at the corners of a sensing plate a male-pattern flow tends to activate just one or two sensors whereas a female pattern flow tends to be more distributed, activating more or all the sensors. Where a male-pattern flow is detected signals from non-activated sensors (sensors with outputs less than a threshold level) may be disregarded. Distinguishing genders can be helpful, for example in distinguishing or ruling out a prostate-related condition.

Optionally urine flow may be sensed using a multiple-axis, for example 3-axis accelerometer, accelerometer data from these multiple axes being combined to determine a parameter reflecting the urine flow rate (helping to make the device flow-direction-insensitive). For example the accelerometer output may be integrated over each of the three (X, Y and Z) directions.

Optionally, multiple axis accelerometer data may be employed to distinguish the gender of the source of the urine flow, by determining one or more characteristics of the accelerometer data and using this to distinguish between genders. For example, the standard deviation of the measured acceleration along one or preferably multiple axes, for example X, Y and/or Z axes, may be employed to distinguish between male and female flows. Optionally the flow rate may be employed to determine an age bracket of the flow originator; alternatively if a device is to be used by a particular age demographic, such as the elderly, this information may be pre-programmed into the device in order to set thresholds and the like.

The system may comprise at least two sensors, a first sensor to detect initiation of the flow and a second sensor to detect flow rate. Operation of the second sensor may be initiated by the first sensor detecting initiation of the flow. Thus the first sensor may comprise a temperature sensor or, in some preferred embodiments, a pressure sensor, and this may be employed to wake up the device/second sensor when flow begins. Alternatively, the first sensor may comprise, for example, an optical sensor such as a colourimetric sensor. Additionally or alternatively, the second sensor may comprise a microphone or similar acoustic vibration sensor. In this case, it is advantageous only to turn on, or begin collecting data from, the acoustic sensor once flow has been detected by, for example, a detected change in pressure and/or change in temperature. This helps to avoid the sensor detecting background noise. It appears that an acoustic sensor is able to detect flow with increased accuracy. It will be appreciated that it is not essential to control operation of an acoustic sensor with another sensor.

As previously mentioned, in embodiments the device is configured to distinguish and disregard the final part of the urine flow, for example based upon the flow pattern and/or flow rate. This helps to make the infection detection more robust. One way in which the final part of the flow can be detected is by the typically reduced pressure of this portion of the flow. Another technique is by the flow pattern, which is somewhat gender-specific - in females the flow apparently ceases whereas in males there is a stop-flow-stop pattern which can be distinguished from the previously mentioned intermittent flow.

As previously mentioned, optionally, examples of the device may sense parameters of the urine such as temperature and/or colour using a temperature and/or optical sensor. A temperature sensor can be employed to determine whether the urine temperature (which reflects the body temperature) is too high or too low (elevated or depressed). An optical sensor can be employed to determine whether or not there is blood in the urine or, more generally, whether the urine is overly dark. Data from one or more such further sensors may be combined with data from the flow rate sensing, for example to indicate infection when any of these conditions is detected, or to indicate infection only when more than one of these conditions is present. Although, generally, the sensing device detects urinary tract infections, other infections such as kidney infections or kidney stones, may also be detected.

Embodiments of the system may include a processor coupled to the sensor device and to memory storing processor control code. The processor control code may comprise code to control the processor to process the input data as described. However in some preferred embodiments the processor includes some dedicated hardware to process the data, and thus some or all of the processing may be implemented in such hardware.

Thus embodiments of the system may include a processor in combination with working memory and non-volatile memory including memory storing processor control code to implement the above described functions. The processor(s) employed in the device may operate under the control of stored program code, or may comprise dedicated hardware implemented in electronic circuitry, or may comprise a combination of some dedicated hardware modules and some systems under program control. Additionally or alternatively, however, the data processing described above may be implemented partially or wholly in dedicated hardware such as a programmable gate array and/or ASIC (application specific integrated circuit). As the skilled person will be aware, the functionality of such a device may be distributed between multiple hardware elements in wired or wireless communication with one another; some or all processing may be performed in hardware, some or all processing may be performed in software.

The disclosure further provides processor control code to implement the above-described devices and methods, for example on a general purpose computer system or on a mobile device, or on a digital signal processor (DSP). The code is provided on a non-transitory physical data carrier such as a disk, CD- or DVD-ROM, programmed memory such as non-volatile memory (eg Flash) or read-only memory (Firmware). Code (and/or data) to implement embodiments of the disclosure may comprise source, object or executable code in a conventional programming language (interpreted or compiled) such as C, or assembly code, or code for a hardware description language. As the skilled person will appreciate such code and/or data may be distributed between a plurality of coupled components in communication with one another.

### BRIEF DESCRIPTION OF THE DRAWINGS

These and other aspects of the invention will now be further described, by way of example only, with reference to the accompanying figures, in which:
Figures 1a to 1c show, respectively, a block diagram of a infection sensing device according to a first example, a vertical cross section through a physical embodiment of the device, and a schematic vertical cross section through a infection sensing device according to a second example;
Figure 2 shows a flow diagram of a procedure for controlling a device of Figure 1;
Figure 3 shows a block diagram of a urine infection sensing device according to an example;
Figure 4 shows a flow diagram of a procedure for controlling the device of Figure 3;
Figures 5a and 5b show preferred embodiments of a urine flow sensor device and its mounting;
Figure 6 shows a block diagram of a urine-flow based diagnostic system according to an embodiment of the invention; and
Figure 7 shows a flow diagram illustrating operation of a urine-flow based diagnostic system according to an embodiment of the invention.

### DETAILED DESCRIPTION OF PREFERRED EMBODIMENT

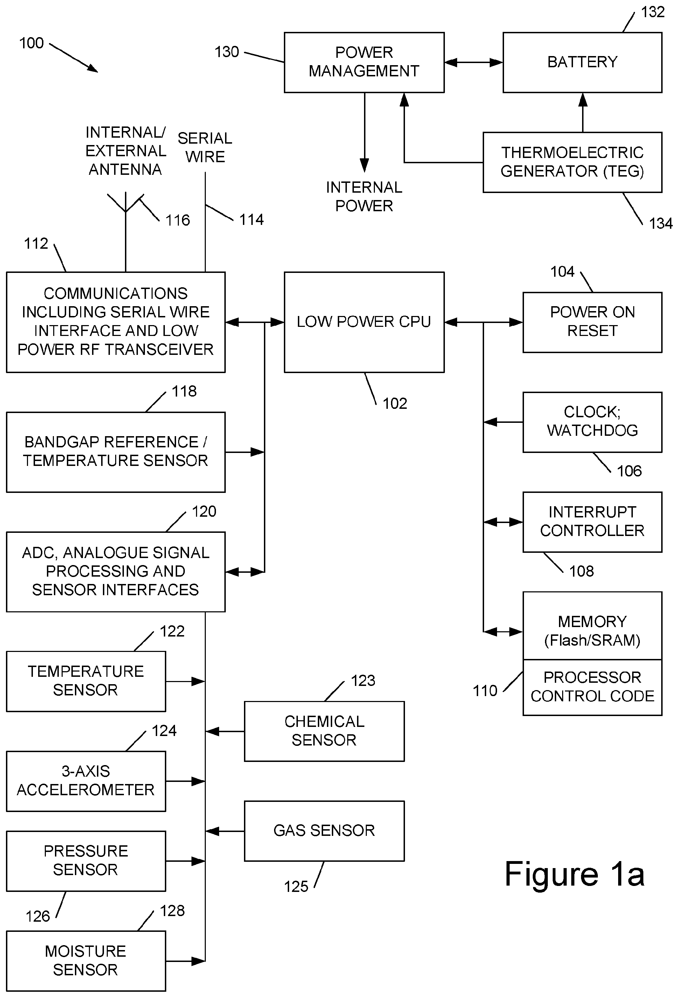

Referring to Figure 1a, this shows an example of a wearable infection sensing device 100. The device comprises a low power CPU 102 coupled to power on reset circuitry 104, to a clock (and to watchdog clock circuitry) 106, to an (optional) interrupt controller 108, to working memory (RAM), and to non-volatile memory (Flash) 110 storing processor control code for controlling CPU 102. The CPU is also coupled to a communications interface 112 including a serial wire interface 114 and/or a low power RF transceiver coupled to an internal and/or external antenna 116. CPU 102 is also coupled to a temperature sensor 108, for example employing a bandgap reference, and to an analogue-to-digital convertor and signal processing interface 120 for interfacing to a set of sensors. These sensors comprise, in embodiments, a further temperature sensor 122, a chemical sensor 123, a 3-axis accelerometer 124, a gas sensor 125, a pressure sensor 126, and a moisture sensor 128. Power management circuitry 130 is coupled to a battery (or supercapacitor) 132, and to a thermoelectric power generator (TEG) 134 or other energy harvester. In operation thermoelectric generator 134 charges battery 132 which provides internal power to the device based on a small difference between the body temperature and environment temperature.

The gas sensor 125 may be a chemical gas sensor and/or an electrochemical nose (e-nose). An example of a gas sensor is the Figaro TGS2602-B00 Air Contaminants Gas Sensor. The gas sensor 125 may be configured to detect the concentration of one or more chemicals in gas in or around the toilet bowl or urinal. The chemical sensor 123 may be configured to detect the concentration of one or more chemicals within the urine. This may be through one or more receptors configured to interact with one or more chemicals to detect the one or more chemicals and measure the associated concentration.

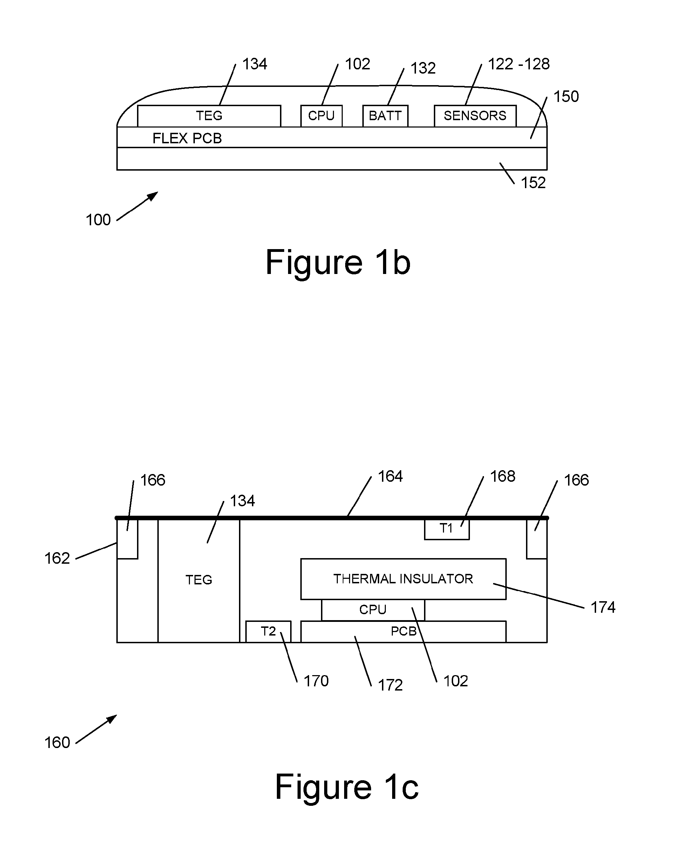

Figure 1b shows a cross section through a physical embodiment of the device, in the illustrated example fabricated on flexible circuit board 150 bearing an adhesive layer 152 so that the device can be attached to the skin, preferably adjacent to an artery. The device includes a CPU, battery, sensors and thermoelectric generator as previously described, preferably covered by a conformal coating 154. Preferably as many of the components as practical are flexible including, for example, the thermoelectric generator and battery. The gas sensor 125 may be at least partially exposed to the surrounding air. The chemical sensor 123 may be exposed to the skin of the subject.

Figure 1c shows a cross section through a further example device 160. In this device an enclosure 162 has a front plate or membrane 164 (typical thickness ~1mm), which is pressure sensitive and can thus be used to detect a heartbeat. To detect pressure the plate is mounted on an accelerometer-spring combination at each corner 166. A first temperature sensor T1 168 is mounted on (thermally coupled to) this plate to measure skin/body temperature and a second temperature sensor T2 179 is mounted on the opposite (external) face of the device, to measure an environmental temperature. This enables compensation for the environmental temperature. A PCB 172 mounts a CPU 102 and other electronics and, importantly, is thermally insulated from sensor T1 by insulation 174. In embodiments a thermoelectric generator 134 spans the skin-contacting and external faces of the device. Optionally a skin moisture sensor (such as one or more electrodes, not shown) may be provided on plate 164. Optionally, a chemical sensor (not shown) may be provided on plate 164. Optionally, a gas sensor (not shown) may be provided on the opposite (external) face of the device (to the plate 164) to measure the concentration of one or chemicals in the surrounding air.

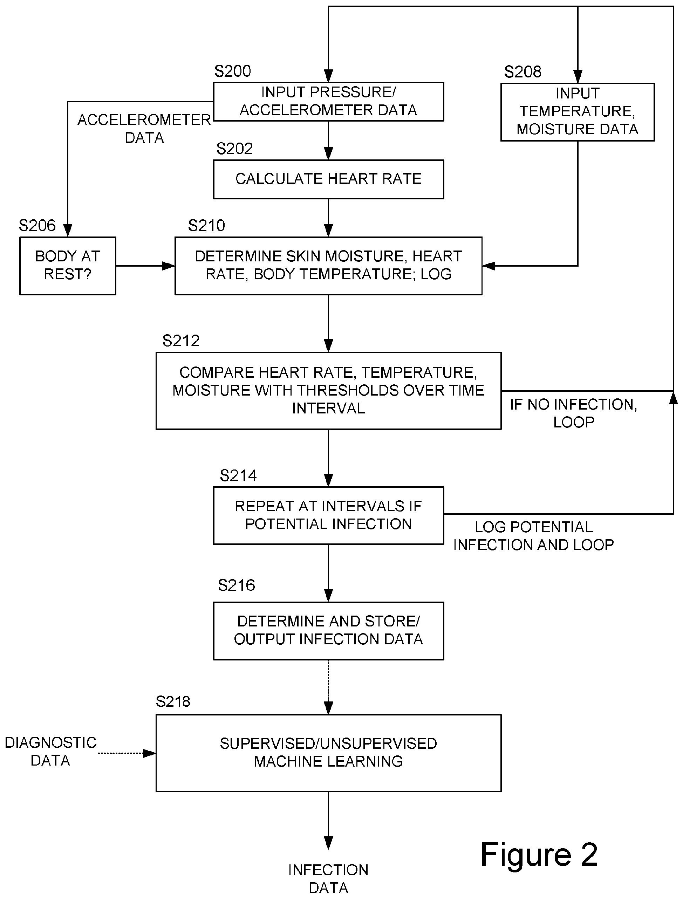

Referring next to Figure 2, this shows a flow diagram of an embodiment of a procedure for the CPU of the wearable device of Figure 1. Thus at step S200 the procedure inputs pressure and accelerometer data and combines this data to calculate a heart rate for the wearer (S202). The procedure also uses the accelerometer data to determine whether or not the person is at rest (S206), disregarding data collected when the person is moving and, preferably, for some duration after the person has stopped moving.

The procedure also collects data from the temperature sensors and humidity sensor (S208) and processes the temperature sensor data to determine an estimated body temperature, and processes the humidity sensor data to determine an estimated skin moisture level (S210); at this point the procedure also has the heart rate data. In embodiments, the raw and/or processed sensor data may optionally be logged for later interrogation. The procedure then applies criteria to the heart rate, temperature and moisture levels to determine whether or not there is a potential infection (S212).

In further embodiments, chemical concentration data (such as from a chemical or gas sensor) may also be input at step S208 and compared to a respective threshold in step S212 to determine whether there is a potential infection.

If no potential infection is identified the procedure loops back to re-check the sensor data; if there is a potential infection the procedure logs the potential infection and loops back (S214) to repeat the test at intervals, for example, every few hours.

At step S212 embodiments of the procedure identify the combination of a temperature fluctuation between high and low temperature values in combination with a raised heart rate, where the elevated heart rate may be determined with respect to an absolute threshold in beats per minute (bpm) or with respect to a heart rate dependent upon an individual or category of individual, for example an age-dependent heart rate. Optionally multiple combinations of heart rate and temperature fluctuations may be identified. For example, a first set of conditions may comprise detecting, during a 3 hour period, that the heart rate goes above 71 bpm on at least one occasion and that there is at least one high-low temperature fluctuation (whilst the body is in its rest state). Such a condition may determine infection with a first probability, for example 75%. A second set of conditions may, for example, define that during a period of 1 hour the heart rate at some point exceeds 75 bpm and there is a high-low temperature fluctuation, again whilst the body is in its rest state; this may define a higher probability of infection, for example 88%. A requirement for an increased skin moisture level may also be included. A third example condition may detect an increased heart rate and an increased moisture level whilst the body temperature is approximately normal; a fourth type of condition may identify a temperature fluctuation without requiring an increased heart rate or increased skin moisture level. One or more of these types of condition may be employed; preferably all are determined when the body is in its rest state.

Once infection data has been determined this data is stored and at some convenient time output on a wired or wireless connection (S216). The infection data may comprise a single bit defining whether or not an infection has been identified, and/or it may include information identifying a level and/or probability of infection and/or it may include data identifying a type of infection or set of conditions identified (as described above), and/or the data may include raw or processed sensor data, for example so that a medical practitioner may review the historically measured heart rate and/or temperature and/or moisture data.

Optionally (un)supervised machine learning may be applied to the raw and/or processed data and/or output data (S218) to provide a more robust indication of potential infection. The machine learning may be implemented on the device of Figure 1a, 1b or 1c, or partially or wholly remotely, for example on a base station or remote server.

Whilst the above embodiments discuss infection sensing, alternative embodiments may be configured to detect a medical condition. For instance, an increase in glucose measured by a chemical sensor may indicate diabetes. Equally, the processor may be configured to detect other medical conditions, such as cancer, through the detection of associated chemicals (e.g. one or more expressions of genes associated with cancer).

The infection sensing system may be configured to reset measurements after use.

This allows the system to be used with a different user. The reset ensures that measurements from a previous user are not erroneously associated with a new user. The reset may be in response to the system being removed from the skin of a user (identified by changes in the detected skin moisture, temperature and pressure). Alternatively, the reset may be via using a magnetic wave from a magnetic tag, and RFID signal from an RFID tag, from a wireless signal (e.g. a Bluetooth signal or a command from a wireless base station), or from a reset input such as a button.

As previously described, the device itself may provide a signal or may communicate by means of a wired or wireless connection with a base unit or base station, for example a mobile phone, desktop computer or the like. The skilled person will appreciate that, in the various different aspects infection data may be provided via a network such as a mobile phone network or the internet, for example to alert a carer. Additionally or alternatively, an alarm may be triggered should an infection level greater than the threshold and/or a persistent infection be detected.

### Urine-based sensing

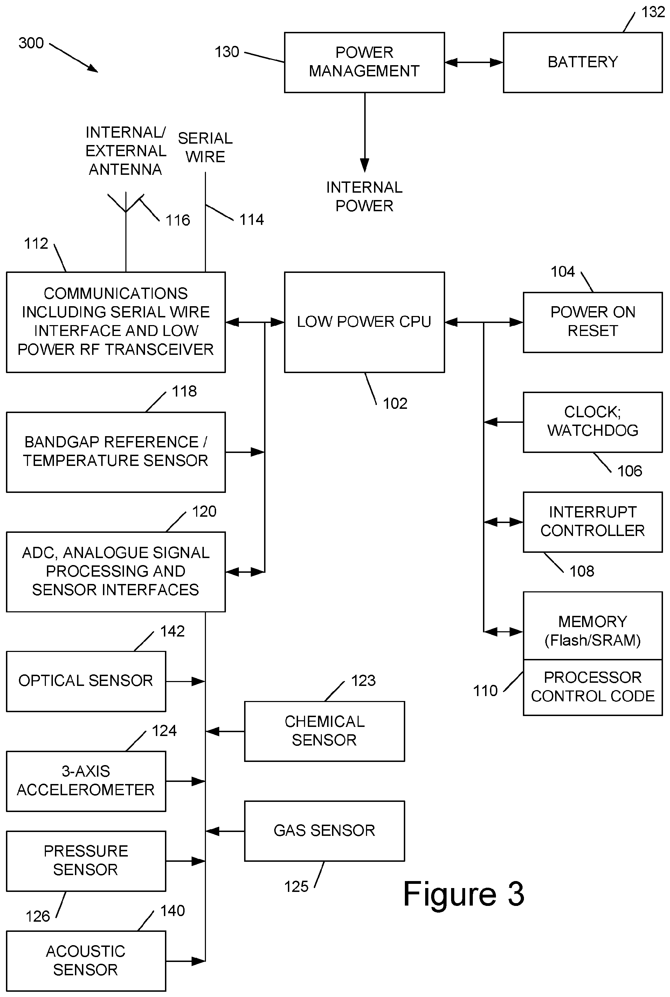

Figure 3 shows a block diagram of a urine sensing device 300, in which like elements to those of Figure 1 are indicated by like reference numerals. As can be seen, a different set of sensors may be employed including, for example, an optional acoustic sensor or microphone 140 and an optional optical sensor 142 for example for colourimetric measurements. The stored processor control code will also be different to that of Figure 1. The device of Figure 3 may be powered by a rechargeable or replaceable battery or may be wireless-chargeable.

Figure 3b illustrates an example physical unit illustrating that the circuitry may be contained in a small plastic case 350, sealed so that it is waterproof, and bearing a hook 352 or some other mounting means to facilitate mounting unit within a toilet bowl or urinal.

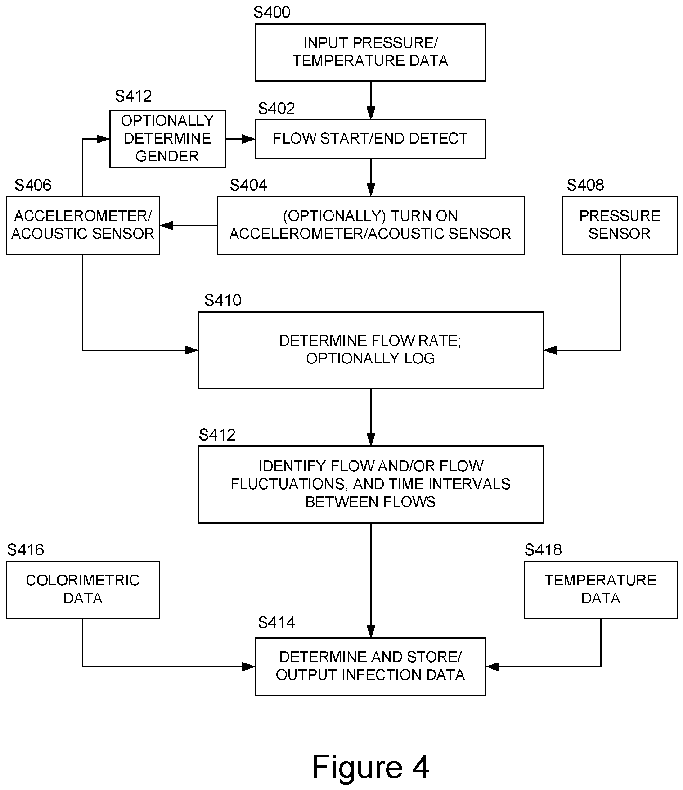

Figure 4 shows a flow diagram of an example procedure implemented by the device at Figure 3. Thus at step 400 the procedure inputs pressure and/or temperature data and uses this to detect the start of a urine flow onto the device (S402). The time at which the urine flow starts may be detected and recorded. This can be used to identify a medical condition, for instance, based on frequency of urination or frequency of urination during a predefined period of time (time of day/night).

Once flow has been detected the procedure begins gathering data from the accelerometer and/or turns on the acoustic sensor (microphone), S404. Data is then collected from the accelerometer and/or acoustic sensor (S406) and, in embodiments, also from the pressure sensor (S408). These data are used to determine the urine flow rate (S410), for example by integrating the X, Y and Z-axis accelerometer signals to determine a direction-insensitive flow rate and then optionally combining this, for example by weighted average, with a flow rate from the pressure sensor, to determine time series flow rate data.

Optionally the accelerometer data may also be provided to a routine S412 which estimates the gender, for example from the standard deviation of the X-,Y- and Z-accelerometer signal components (greater for males than for females). This information may provide information to the flow-end detection procedure S402 as the male and female end of flow behaviour differs. In broad terms, therefore, embodiments of the device may estimate the user's gender, and then adapt the operation of the device in response to the user's gender, in particular to increase the accuracy of the data obtained. Although in embodiments this information is applied to end of flow detection a skilled person will appreciate that knowledge of gender may be used in other ways to improve the accuracy of the measurements.

Once flow rate has been determined, the procedure identifies the absolute flow rate as a low flow rate, for example less than 10 ml/s, can indicate infection (S412). The procedure identifies flow fluctuations and counts the number of peaks in the flow. In some preferred embodiments the procedure identifies when the flow falls to substantially zero, and preferably also determines a duration or an average duration between such intervals and/or counts a number of such intervals. In particular, the duration between points at which the flow approaches zero is useful for identifying a potential infection. More particularly, where an infection is present there may be an extended period of time for which the flow is close to zero (below a threshold level). Thus there may be short intervals of flow punctuated by relatively long delays. In embodiments, therefore, the device may time the duration of one or more of such delays between intervals of flow and determine a delay or an average delay. In broad terms, the longer the delay between intervals of flow the greater the likelihood of infection and/or greater the degree of infection. The skilled person will appreciate that there are many ways to define a threshold level of flow in order to count or time durations of low flow or intervals between duration periods of flow.

In further embodiments, a gas and/or chemical sensor is/are turned on at step S404 and measure chemical concentration at step S406. This concentration data is input in step S410 to determine whether there is a potential infection.

At step 414 the procedure determines and stores/outputs infection data. As previously, this may be a simple binary bit indicating the presence or absence of potential infection. However more information may be provided, for example information indicating a degree of infection, a probability of infection, a type of potential infection, infection characterising data, raw and/or processed sensor data, and so forth. In particular, the device may include an optical sensor to provide colourimetric data (S416) and/or a temperature sensor to provide temperature data (S418).

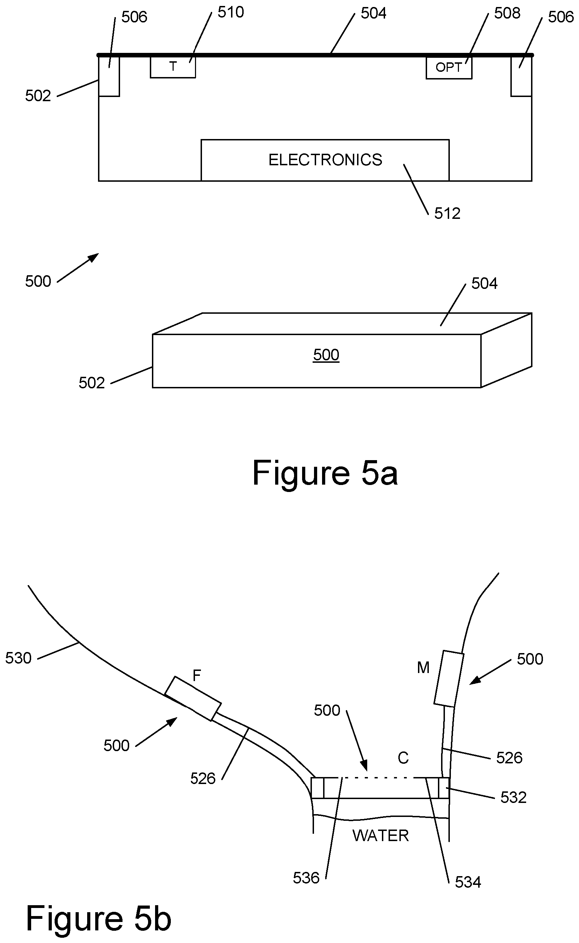

Referring now to Figure 5a, this shows a preferred embodiment of a sensing device 500, comprising an enclosure 502 with a pressure-sensitive front plate or membrane 504, similar to that previously described with reference to Figure 1c. The plate 504 is mounted on a set of pressure sensors 506, for example one at each corner of the enclosure. The device includes electronics 512, including communications and a power supply. The device may include an optical (urine colour/optical density) sensor 506 (in which case the plate 504 may be clear), and a temperature sensor 510. A perspective view of the device is also shown.

The device may further include a chemical sensor and/or a gas sensor (not shown). The chemical/gas sensors may be at least partially exposed through the casing.

Figure 5b shows how one or more sensor devices 500 may be positioned within a toilet bowl 530. Position M is suited to male use; position F to female use; central position C is suitable for both. Sensors at M and F may be glued onto the toilet bowl.

The sensor device shown in Figure 5a is rectangular but for use in position C the sensor device may have a ring-shaped enclosure 532 mounting a plate 534 spanning the ring and having apertures to allow urine to pass through. Where multiple sensor devices are present the sensor at position C may be suspended by linkages 526. One or more of these may be magnetic, and hence detachable; a linkage may also provide an electrical connection. Where multiple sensor devices are present they may be in wired or wireless communication with one another.

The sensor device for use in position C may be mounted on a pivoting mechanism and may include a motor to move the enclosure 532 up, away from the water. A cleaning system may be located above the sensor device. The cleaning system may comprise a reservoir and may be configured to release fluid (e.g. cleaning fluid) to clean the sensor device (e.g. flush away waste). This is because the conventional flush mechanism in a toilet may not be sufficient to remove contaminants from the sensors. The pivoting mechanism may allow cleaning fluid to be collected over the sensors to improve the cleaning action. Once the sensing device has been cleaned, the pivoting mechanism may return the sensing device to its previous position (position C).

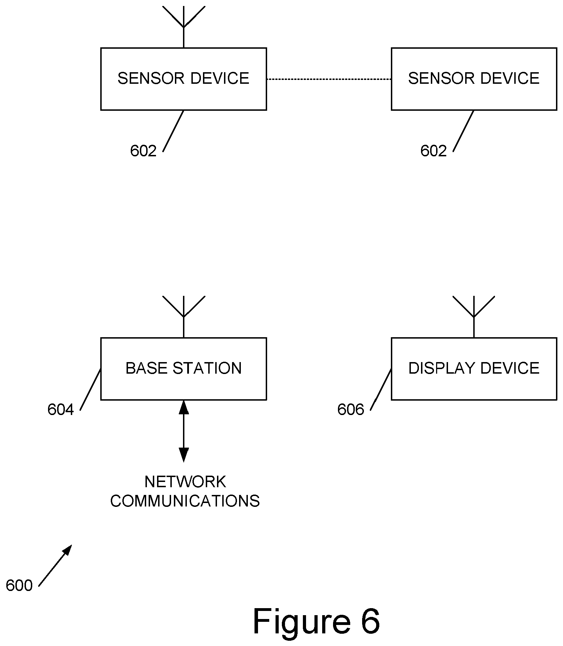

Figure 6 illustrates a block diagram of an example urine-flow based diagnostic system 600. The system comprises one or more sensor devices 602, coupled to a base station 604 and/or to a local display device 606 to provide alerts and/or sensed data, and the like. The system may be coupled to the cloud via a network.

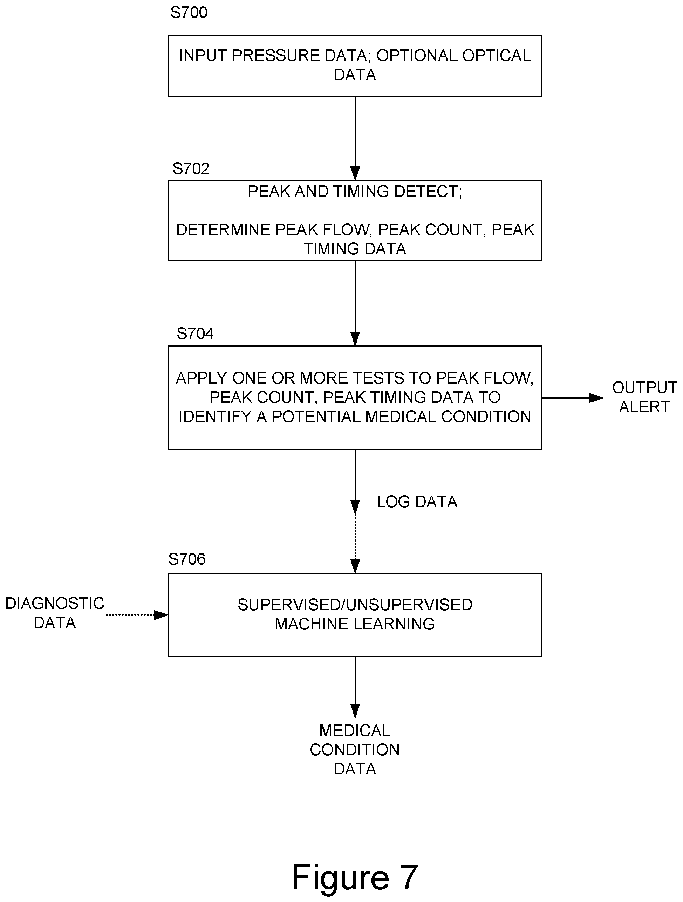

Figure 7 shows a flow diagram illustrating operation of a urine-flow based diagnostic system according to a preferred embodiment of the invention.

At S700 the procedure captures pressure data as a proxy for urine flow rate, and optionally data for an optical measurement of urine colour/opacity. In embodiments the pressure data may be converted to a flow rate in ml/sec based upon a calibration (which may be linear or non-linear). Although shown as a single step data are captured at intervals of a few milliseconds; the system may be woken up automatically to collect data.

The procedure performs peak and peak-timing detect S702, in one implementation by first smoothing the input data (e.g. filtering in the time domain), and then storing the captured data in a time-series array. A peak may be detected by matching values to either side of a detected maximum; this can be used to determine the duration of a peak. An inter-peak duration may be determined by matching values to either side of a detected minimum. For example in one embodiment the flow rate values in ml/sec are quantised to integer values and then values to either side of a minimum identified (e.g. if the minimum is 2ml/sec an inter-peak duration may be determined from the interval between flow rates of 3ml/sec). Once peaks have been identified they may be counted and an inter-peak timing determined, as well as a maximum flow rate for the peak. In embodiments flow rates below a threshold (e.g. 0.2ml/sec) are disregarded; in embodiments the final two peaks identified are also discarded.

The procedure then processes that data to determine signals of potential infection and of a potential prostate condition (S704). Test as previously described in the Summary of the Invention may be employed. In embodiments multiple signals are required to be present in combination for a positive detection of a potential medical condition. Test applied may include one or more of: Detection of multiple peaks; detection of a low flow rate (<10ml/sec or <5ml.sec); detection of an extended time between peaks (>0.5sec) for infection; detection of less than a threshold or zero time between peaks for a prostate condition; detection of greater than a threshold peak duration (e.g. greater than 10sec, 15 sec or 20 sec) for a prostate condition.

A result of this processing may be stored and/or output as data indicating the presence of a potential medical condition and, optionally, whether the condition is an infection or a prostate-related condition. Optionally a potential degree of severity of the condition may also be indicated. Additionally or alternatively the raw data from S700 and/or processed data from S702 may be provided. In embodiments an alert and/or the raw/processed data may also be provided on display device 606 and/or communicated to a remote server for further storage/processing.

In embodiments data from one or more of steps S700, S702 is provided to a machine learning procedure S706, for example running in base station 604 and/or on a remote server. The procedure may implement a supervised or unsupervised learning algorithm, for example using supervised learning in combination with data inputted from diagnoses previously made using data from the system, or using unsupervised learning to classify the input data. The machine learning procedure may thus learn to provide improved data indicating the presence of a potential medical condition, and/or differentiation of one or more conditions (such as infection and prostate-related conditions).

Further parameters may be utilised to detect infections or medical conditions. These parameters may include chemical concentration (either in urine or in gas emanating from the urine) and time or frequency of urination.

Whilst the above embodiments discuss the detection of an infection, embodiments may be configured to detect other medical conditions, such as cancer, kidney stones or diabetes. For instance, diabetes may be detected through increased frequency of urination at night and/or increased glucose or ketone concentrations in urine. Equally, cancer, such as prostate cancer, may be detected through an increased concentration of chemicals associated with genes associated with the cancer (e.g. PSA and expressions of TMPRSS2:ERG or PCA3).

As previously described, the device itself may provide a signal or may communicate by means of a wired or wireless connection with a base unit or base station, for example a mobile phone, desktop computer or the like. The skilled person will appreciate that, in the various different aspects of the invention, the infection data may be provided via a network such as a mobile phone network or the internet, for example to alert a carer. Additionally or alternatively, an alarm may be triggered should an infection level greater than the threshold and/or a persistent infection be detected.

The urine-flow based diagnostic system may be configured to connect to one or more further sensing devices. For instance, the urine sensing device may receive measurements taken by the infection sensing systems described with reference to Figures 1-3, and vice versa. One device may be configured to trigger measurements in the other. For instance, the infection sensing system may take measurements (e.g. heart rate, skin temperature, skin moisture, etc.) in response to a signal from the urine-flow based diagnostic system that the flow has started (indicating that the user is urinating). Measurements from both devices may therefore be combined for improved identification of medical conditions. The measurements may be processed in one of the devices or may be exported to an external system (e.g. a computer) for processing. Accordingly, whilst the above embodiments are described as diagnostic systems, embodiments may not perform any analysis, and may instead report measurements to an external system for analysis.

The urine-flow based diagnostic system may be configured to reset measurements after use. This reset ensures that measurements from a previous user are not erroneously associated with a new user. The reset may be in response to the system being flushed (either by an external flushing mechanism or via the cleaning mechanism described above). Alternatively, the reset may be via using a magnetic wave from a magnetic tag, an RFID signal from an RFID tag, from a wireless signal (e.g. a Bluetooth signal or a command from a wireless base station), or from a reset input such as a button or pull-chord.

The systems described herein may associate measurements with users through the use of a unique user identifier. This may be input via a command from an external system (e.g. via a wireless interface), or may be input from an RFID tag or magnetic tag that the system is configured to read. This allows the system to be used with different users, and for measurements to be taken over time and associated with the respective user. This may be particularly advantageous for the urine-flow based diagnostic system (although it may be implemented by the infection system of Figures 1-3) as multiple users may use the system when it is fitted to a toilet or urinal.

The processing has been described as taking place at the sensing device but the skilled person will appreciate that in embodiments the processing may be partly or substantially wholly at a mobile or fixed computing device with which the sensing device communicates. Thus the device may transmit sensor data, for example via a Bluetooth or other link, to a mobile device implementing a procedure as described above. Alternatively the processing may be distributed, partly on the sensing device and partly on, say, a mobile device.

No doubt many other effective alternatives will occur to the skilled person. It will be understood that the invention is not limited to the described embodiments and encompasses modifications apparent to those skilled in the art lying within the scope of the claims appended hereto.

## Claims

1. A urine-flow based diagnostic system (600), the system (600) comprising:
a sensor device (500) configured to be used in a toilet bowl or urinal, the sensor device being further configured to intersect a flow of urine, the sensor device (500) comprising a urine stream flow sensor; and
a processor (102) coupled to said urine stream flow sensor; and
wherein the processor (102) is configured to:
determine, from said urine stream flow sensor, a flow rate parameter dependent on the sensed urine flow
identify presence of a potential medical condition based on the presence of a plurality of peaks in said flow rate parameter;
distinguish between the presence of a prostate condition and an infection dependent upon a number of said detected peaks and a flow rate of said stream of urine; and
responsive to said flow rate parameter, store and/or output data indicating the presence of the potential medical condition.

2. A system (600) as claimed in claim 1 wherein said processor (102) is further configured to identify presence of a potential medical condition based on the identification of a flow of said stream of urine as intermittent based on said flow rate.

3. A system (600) as claimed in claim 1 wherein said processor (102) is configured to characterise a timing of said peaks to identify presence of potential infection.

4. A system (600) as claimed in claim 3 wherein characterisation of said timing comprises one or more of:
determining a time duration, T1, between one or more of said peaks;
determining a time duration, T2, of a period of reduced or substantially zero flow between one or more of said peaks;
determining a time duration, T3, of one or more of said peaks;
determining a ratio between T2 and T3,
wherein said characterisation comprises identifying presence of potential infection by identifying when T2 is increased or above a threshold value and/or T3 is reduced or below a threshold value.

5. A system (600) as claimed in any preceding claim wherein:
said processor (102) is configured to identify when a peak flow rate is less than a threshold to identify presence of said potential medical condition, in particular identify the potential presence of a prostate condition dependent upon a flow rate range of said stream of urine; and/or
said sensor device (500) further comprises a urine stream colour sensor (508); and wherein said processor (102) is further configured to identify presence of the potential medical condition responsive to a detected degree of colouration of said urine.

6. A system (600) as claimed in any preceding claim wherein:
said sensor device (500) further comprises a chemical sensor (123), and said processor (102) is further configured to identify presence of the potential medical condition responsive to a detected concentration of a chemical indicative of a medical condition within said urine; and/or
said sensor device (500) further comprises a gas sensor (125) and said processor (102) is further configured to identify presence of the potential medical condition responsive to a detected concentration of a chemical indicative of a medical condition in gas deriving from said urine.

7. A system (600) as claimed in any preceding claim wherein said urine stream flow sensor comprises a plurality of spatially distributed flow sensors, and wherein said processor (102) is configured to combine data from said distributed flow sensors to sense said urine flow.

8. A system (600) as claimed in preceding claim wherein said processor (102) is configured to distinguish between genders for said urine flow dependent upon a sensed spatial distribution of said urine flow.

9. A system (600) as claimed in claim 7 or claim 8 wherein said sensor device (500) comprises an enclosure with a sensing surface comprising a moveable plate or membrane (504) mounted on a set of pressure sensors (506), wherein said set of pressure sensors (506) comprises said distributed flow sensors.

10. A system (600) as claimed in any preceding claims further comprising a cleaning system comprising a reservoir configured to hold fluid and a release mechanism for releasing at least some of the fluid to clean at least part of the sensor device (500), wherein the processor (102) is configured to control the release mechanism to release the fluid after urine has been detected.

11. A system (600) as claimed in claim 10 further comprising a fluid collection mechanism configured to collect fluid over one or more sensing apparatus of the system after the fluid has been released from the reservoir.

12. A system (600) as claimed in any preceding claim wherein the processor (102) is configured to record a time at which the urine flow is detected and, responsive to one or more of an increased frequency of urination or an increased frequency of urination during a predetermined period of the day-night cycle, store and/or output data indicating the presence of a medical condition.

13. A system (600) as claimed in any preceding claim wherein the system comprises an input means and wherein the processor (102) is configured to identify the presence of a new user in response to an input from a user and, in response to the identification of the new user, establish a new set of measurements for the new user, wherein the processor (102) is configured to identify the new user in response to receipt of a unique user identifier and to associate any subsequent measurements and identifications of medical conditions with the unique user identifier until the presence of another user is identified.

14. A non-transitory data carrier carrying processor control code for implementation by the urine-flow based diagnostic system of claim 1, wherein the processor control code is configured to cause the processor (102), coupled to a urine stream flow sensor of a sensor device (500) for use in a toilet bowl or urinal that is configured to detect a stream of urine, to:
determine, from said urine stream flow sensor, a flow rate parameter dependent on a sensed urine flow;
identify presence of a potential medical condition based on the presence of a plurality of peaks in said flow rate parameter;
distinguish between the presence of a prostate condition and an infection dependent upon one or both of a number of said detected peaks and a flow rate of said stream of urine;
characterise a timing of said peaks to identify presence of potential infection; and responsive to said flow rate parameter, store and/or output data indicating the presence of the potential medical condition.

## Patentansprüche

1. Auf Urinstrommessung basierendes Diagnosesystem (600), wobei das System (600) Folgendes umfasst:
eine Sensorvorrichtung (500), die zur Verwendung in einer Toilettenschüssel oder einem Urinal konfiguriert ist, wobei die Sensorvorrichtung ferner so konfiguriert ist, dass sie einen Urinstrom schneidet, wobei die Sensorvorrichtung (500) einen Urinstromsensor umfasst; und
einen Prozessor (102), der mit dem Urinstromsensor gekoppelt ist; und
wobei der Prozessor (102) konfiguriert ist, zum:
Bestimmen, mithilfe des genannten Urinstromsensors, eines Durchflussparameters, der vom gemessenen Urinstrom abhängt
Identifizieren des Vorhandenseins einer potenziellen Erkrankung anhand des Auftretens einer Vielzahl von Spitzenwerten im Durchflussparameter;
Unterscheiden zwischen einer Prostataerkrankung und einer Infektion anhand einer Anzahl der erfassten Spitzen und der Urinstromdurchflussrate; und
als Reaktion auf den Durchflussparameter, Speichern und/oder Ausgeben von Daten, die auf das Vorliegen des potenziellen Erkrankung hinweisen.

2. System (600) nach Anspruch 1, wobei der Prozessor (102) ferner so konfiguriert ist, dass er Vorliegen einer potenziellen Erkrankung anhand der Bestimmung erkennt, dass der Urinstrom intermittierend ist, basierend auf der Durchflussrate.

3. System (600) nach Anspruch 1, wobei der Prozessor (102) so konfiguriert ist, dass er die zeitliche Abfolge der Spitzenwerte charakterisiert, um das Vorhandensein einer potenziellen Infektion zu identifizieren.

4. System (600) nach Anspruch 3, wobei die Charakterisierung der genannten zeitlichen Abfolge eines oder mehrere der Folgenden umfasst:
Bestimmen der Zeitdauer T1 zwischen einem oder mehreren der Spitzenwerte;
Bestimmen der Zeitdauer T2 eines Zeitraums mit reduziertem oder im Wesentlichen keinem Durchfluss zwischen einem oder mehreren der Spitzenwerte;
Bestimmen der Zeitdauer T3 eines oder mehrerer der Spitzenwerte;
Bestimmen des Verhältnisses zwischen T2 und T3,
wobei die Charakterisierung Identifizieren des Vorhandenseins einer potenziellen Infektion umfasst, indem identifiziert wird, wann T2 erhöht ist oder einen Schwellenwert überschreitet und/oder T3 reduziert ist oder einen Schwellenwert unterschreitet.

5. System (600) nach einem vorstehenden Anspruch, wobei:
der Prozessor (102) so konfiguriert ist, dass er identifiziert, wenn eine Spitzendurchflussrate unter einem Schwellenwert liegt, um Vorliegen der genannten potenziellen Erkrankung festzustellen, insbesondere das mögliche Vorliegen einer Prostataerkrankung in Abhängigkeit von einem Durchflussbereich des Urinstroms zu identifizieren; und/oder
die Sensorvorrichtung (500) ferner einen Urinstromfarbsensor (508) umfasst; und wobei der Prozessor (102) ferner so konfiguriert ist, dass er Vorhandensein einer potenziellen Erkrankung identifiziert, als Reaktion auf einen bestimmten Farbton des Urins.

6. System (600) nach einem vorstehenden Anspruch, wobei:
die Sensorvorrichtung (500) ferner einen chemischen Sensor (123) umfasst, und der Prozessor (102) ferner so konfiguriert ist, dass er das Vorhandensein einer potenziellen Erkrankung identifiziert, als Reaktion auf eine festgestellte Konzentration einer chemischen Substanz im Urin, die auf eine Erkrankung hinweist; und/oder
die Sensorvorrichtung (500) ferner einen Gassensor (125) umfasst, und der Prozessor (102) ferner so konfiguriert ist, dass er das Vorhandensein der potenziellen Erkrankung identifiziert, als Reaktion auf eine detektierte Konzentration einer chemischen Substanz, die auf eine Erkrankung hinweist, in Gasen, die aus dem Urin stammen.

7. System (600) nach einem vorstehenden Anspruch, wobei der Urinstromsensor eine Vielzahl räumlich verteilter Durchflusssensoren umfasst und wobei der Prozessor (102) so konfiguriert ist, dass er Daten von den verteilten Durchflusssensoren kombiniert, um den Urinstrom zu erfassen.

8. System (600) nach einem vorstehenden Anspruch, wobei der Prozessor (102) so konfiguriert ist, dass er anhand der erfassten räumlichen Verteilung des Urinstroms zwischen den Geschlechtern unterscheidet.

9. System (600) nach Anspruch 7 oder 8, wobei die Sensorvorrichtung (500) ein Gehäuse mit einer Sensorfläche umfasst, die eine bewegliche Platte oder Membran (504) umfasst, die auf einem Satz von Drucksensoren (506) montiert ist, wobei der Satz von Drucksensoren (506) die verteilten Durchflusssensoren umfasst.

10. System (600) nach einem vorstehenden Anspruch, ferner umfassend ein Reinigungssystem mit einem Reservoir konfiguriert zur Aufnahme von Flüssigkeit und einem Freigabemechanismus zum Freisetzen von mindestens einem Teil des Fluids zur Reinigung mindestens eines Teils der Sensorvorrichtung (500), wobei der Prozessor (102) so konfiguriert ist, dass er den Freigabemechanismus steuert, um das Fluid freizusetzen, nachdem Urin erkannt wurde.

11. System (600) nach Anspruch 10, ferner umfassend einen Fluidsammelmechanismus, der so konfiguriert ist, dass er Fluid über einer oder mehreren Sensoreinrichtungen des Systems sammelt, nachdem das Fluid aus dem Reservoir freigesetzt wurde.

12. System (600) nach einem vorstehenden Anspruch, wobei der Prozessor (102) so konfiguriert ist, dass er den Zeitpunkt der Erkennung des Urinstroms aufzeichnet und, als Reaktion auf eine oder mehrere von erhöhter Miktionsfrequenz oder erhöhte Miktionsfrequenz während eines vorbestimmten Zeitraums des Tag-Nacht-Zyklus, Daten, die auf Vorliegen einer Erkrankung hinweisen, speichert und/oder ausgibt.

13. System (600) nach einem vorstehenden Anspruch, wobei das System eine Eingabeeinrichtung umfasst und der Prozessor (102) so konfiguriert ist, dass er die Anwesenheit eines neuen Benutzers als Reaktion auf eine Benutzereingabe erkennt und als Reaktion auf die Identifizierung des neuen Benutzers einen neuen Satz von Messungen für den neuen Benutzer erstellt, wobei der Prozessor (102) so konfiguriert ist, dass er den neuen Benutzer als Reaktion auf den Empfang einer eindeutigen Benutzerkennung identifiziert und alle nachfolgenden Messungen und Identifizierungen von Erkrankungen mit der eindeutigen Benutzerkennung verknüpft, bis die Anwesenheit eines anderen Benutzers festgestellt wird.

14. Nichtflüchtiger Datenträger, der Prozessorsteuerungscode zur Implementierung durch das urinstrombasierte Diagnosesystem nach Anspruch 1 enthält, wobei
der Prozessorsteuerungscode so konfiguriert ist, dass der Prozessor (102), der mit einem Urinstromsensor einer Sensorvorrichtung (500) zur Verwendung in einer Toilettenschüssel oder einem Urinal verbunden ist, das zur Erkennung eines Urinstroms konfiguriert ist, Folgendes ausführt:
Bestimmen, mithilfe des Urinstromsensors, eines Durchflussparameters, der von dem gemessenen Urinstrom abhängt;
Identifizieren des Vorhandenseins einer potenziellen Erkrankung anhand des Auftretens einer Vielzahl von Spitzenwerten im Durchflussparameter;
Unterscheiden zwischen dem Vorliegen einer Prostataerkrankung und einer Infektion anhand einer oder beider von den erfassten Spitzenwerten und einer Durchflussrate des Urinstroms;
Charakterisieren einer zeitlichen Abfolge der Spitzenwerte, um das Vorliegen einer potenziellen Infektion zu identifizieren; und
in Abhängigkeit vom genannten Durchflussparameter, Speichern und/oder Ausgeben von Daten, die auf das Vorliegen der potenziellen Erkrankung hinweisen.

## Revendications

1. Système de diagnostic basé sur un écoulement d'urine (600), le système (600) comprenant :
un dispositif de détection (500) configuré pour être utilisé dans une cuvette de toilettes ou un urinoir, le dispositif de détection étant en outre configuré pour intercepter un écoulement d'urine, le dispositif de détection (500) comprenant un capteur d'écoulement de jet d'urine ; et
un processeur (102) couplé audit capteur d'écoulement de jet d'urine ; et
dans lequel le processeur (102) est configuré pour :
déterminer, à partir dudit capteur d'écoulement de jet d'urine, un paramètre de débit d'écoulement dépendant de l'écoulement d'urine mesuré.
identifier la présence d'une affection médicale potentielle en se basant sur la présence d'une pluralité de pics dans ledit paramètre de débit d'écoulement ;
distinguer la présence d'une affection de la prostate et une infection en fonction du nombre de pics détectés et d'un débit d'écoulement dudit jet d'urine ;
en fonction dudit paramètre de débit d'écoulement, stocker et/ou délivrer en sortie des données indiquant la présence de l'affection médicale potentielle.

2. Système (600) selon la revendication 1, dans lequel ledit processeur (102) est en outre configuré pour identifier la présence d'une affection médicale potentielle en se basant sur l'identification d'un écoulement dudit jet d'urine comme intermittent en fonction dudit débit d'écoulement.

3. Système (600) selon la revendication 1, dans lequel ledit processeur (102) est configuré pour caractériser une synchronisation desdits pics afin d'identifier la présence d'une infection potentielle.

4. Système (600) selon la revendication 3, dans lequel la caractérisation de ladite synchronisation comprend un ou plusieurs des étapes suivantes consistant à :
déterminer une durée, T1, entre un ou plusieurs desdits pics ;
déterminer une durée, T2, d'une période d'écoulement réduit ou sensiblement nul entre un ou plusieurs desdits pics ;
déterminer une durée, T3, d'un ou de plusieurs desdits pics ;
déterminer un rapport entre T2 et T3,
dans lequel ladite caractérisation comprend l'identification de la présence d'une infection potentielle en identifiant le moment où T2 est augmentée ou supérieure à une valeur seuil et/ou T3 est réduite ou inférieure à une valeur seuil.

5. Système (600) selon une quelconque revendication précédente, dans lequel :
ledit processeur (102) est configuré pour identifier lorsqu'un débit d'écoulement de pic est inférieur à un seuil pour identifier la présence de ladite affection médicale potentielle, en particulier identifier la présence potentielle d'une affection de la prostate en fonction d'une plage de débit d'écoulement dudit jet d'urine ; et/ou
ledit dispositif de détection (500) comprend en outre un capteur de couleur de jet d'urine (508) ; et dans lequel ledit processeur (102) est en outre configuré pour identifier la présence de l'affection médicale potentielle répondant à un degré de coloration détecté de ladite urine.

6. Système (600) selon une quelconque revendication précédente, dans lequel :
ledit dispositif de détection (500) comprend en outre un capteur chimique (123), et ledit processeur (102) est en outre configuré pour identifier la présence de l'affection médicale potentielle en réponse à une concentration détectée d'un produit chimique indicatif d'une affection médicale dans ladite urine ; et/ou
ledit dispositif de détection (500) comprend en outre un capteur de gaz (125) et ledit processeur (102) est en outre configuré pour identifier la présence de l'affection médicale potentielle en réponse à une concentration détectée d'un produit chimique indicatif d'une affection médicale dans le gaz provenant de ladite urine.

7. Système (600) selon une quelconque revendication précédente, dans lequel ledit capteur d'écoulement de jet d'urine comprend une pluralité de capteurs d'écoulement spatialement répartis, et dans lequel ledit processeur (102) est configuré pour combiner les données desdits capteurs d'écoulement répartis afin de détecter ledit écoulement d'urine.

8. Système (600) selon la revendication précédente, dans lequel ledit processeur (102) est configuré distinguer les genres pour ledit écoulement d'urine en fonction d'une distribution spatiale détectée dudit écoulement d'urine.

9. Système (600) selon la revendication 7 ou la revendication 8, dans lequel ledit dispositif de détection (500) comprend un boîtier avec une surface de détection comprenant une plaque ou membrane mobile (504) montée sur un ensemble de capteurs de pression (506), dans lequel ledit ensemble de capteurs de pression (506) comprend lesdits capteurs d'écoulement répartis.

10. Système (600) selon une quelconque revendication précédente comprenant en outre un système de nettoyage comprenant un réservoir configuré pour contenir un fluide et un mécanisme de libération pour libérer au moins une partie du fluide afin de nettoyer au moins une partie du dispositif de détection (500), dans lequel le processeur (102) est configuré pour commander le mécanisme de libération afin de libérer le fluide après que l'urine a été détectée.

11. Système (600) selon la revendication 10 comprenant en outre un mécanisme de collecte de fluide configuré pour collecter le fluide sur un ou plusieurs appareils de détection du système après que le fluide a été libéré du réservoir.

12. Système (600) selon une quelconque revendication précédente, dans lequel le processeur (102) est configuré pour enregistrer une heure à laquelle l'écoulement d'urine est détecté et, en réponse à une ou plusieurs d'une fréquence augmentée des mictions ou d'une fréquence augmentée des mictions pendant une période prédéterminée du cycle jour-nuit, stocker et/ou délivrer en sortie des données indiquant la présence d'une affection médicale.

13. Système (600) selon une quelconque revendication précédente, dans lequel le système comprend un moyen d'entrée et dans lequel le processeur (102) est configuré pour identifier la présence d'un nouvel utilisateur en réponse à une entrée d'un utilisateur et, en réponse à l'identification du nouvel utilisateur, établir un nouvel ensemble de mesures pour le nouvel utilisateur, dans lequel le processeur (102) est configuré pour identifier le nouvel utilisateur en réponse à la réception d'un identifiant utilisateur unique et pour associer toutes les mesures et identifications ultérieures d'affections médicales à l'identifiant utilisateur unique jusqu'à ce que la présence d'un autre utilisateur soit identifiée.

14. Support de données non transitoire transportant un code de commande de processeur destiné à être mis en œuvre par le système de diagnostic basé sur l'écoulement d'urine de la revendication 1, dans lequel
le code de commande de processeur est configuré pour amener le processeur (102), couplé à un capteur d'écoulement de jet d'urine d'un dispositif de détection (500) à être utilisé dans une cuvette de toilettes ou un urinoir configuré pour détecter un jet d'urine, à :
déterminer, à partir dudit capteur d'écoulement de jet d'urine, un paramètre de débit d'écoulement dépendant d'un écoulement d'urine détecté ;
identifier la présence d'une affection médicale potentielle en se basant sur la présence d'une pluralité de pics dans ledit paramètre de débit d'écoulement ;
distinguer la présence d'une affection de la prostate et une infection en fonction du nombre de pics détectés et d'un débit d'écoulement dudit jet d'urine ou les deux ;
caractériser une synchronisation de ces pics afin d'identifier la présence d'une infection potentielle ; et
en réponse audit paramètre de débit d'écoulement, stocker et/ou délivrer en sortie des données indiquant la présence de l'affection médicale potentielle.
